# EUROPEAN PATENT SPECIFICATION

(11) **EP 1 461 424 B1**
(45) Date of publication and mention of the grant of the patent: **16.01.2013**
(21) Application number: 02789508.5
(22) Date of filing: 07.11.2002
(51) Int. Cl.: C12N 7/01, C12N 7/02, C12N 7/04, C12N 15/80, C12N 15/87, A61K 39/12

(54) **GENERATION OF VIRUS-LIKE PARTICLES AND DEMONSTRATION OF LIPID RAFTS AS SITES OF FILOVIRUS ENTRY AND BUDDING**
ERZEUGUNG VON VIRUSÄHNLICHEN TEILCHEN UND NACHWEIS VON LIPID-RAFTS ALS EINTRITTSSTELLEN FÜR FILOVIRUS UND KEIMUNG
GENERATION DE PARTICULES SIMILAIRES AUX VIRUS ET DEMONSTRATION DE RADEAUX LIPIDIQUES SOUS LA FORME DE SITES D'ENTREE ET DE BOURGEONNEMENT DE FILOVIRUS

(30) Priority: 07.11.2001 US 338936 P
(43) Date of publication of application: 29.09.2004
(73) Proprietor: U.S. Army Medical Research Institute of Infectious Diseases; Department of the Army, Frederick, MD 21702 (US)
(72) Inventor: BAVARI, Sina, Frederick, MD 21703 (US); AMAN, M., Javad, North Potomac, MD 20878 (US); SCHMALJOHN, Alan, L., Frederick, MD 21702 (US)
(74) Representative: Sexton, Jane Helen
(86) International application number: PCT/US2002/035834
(87) International publication number: WO 2003/039477

(56) References cited:
- US-B1- 6 200 959
- JASENOSKY L.D. ET AL.: 'Ebola virus VP40-induced particle formation and association with the lipid bilayer' JOURNAL OF VIROLOGY vol. 75, no. 11, June 2001, pages 5205 - 5214, XP002976163
- THORBEN LUNDSGAARD: 'Filovirus-like particles detected in the leafhopper Psammotettix alienus' VIRUS RESEARCH vol. 48, November 1997, pages 35 - 40, XP002976164
- NODA T. ET AL.: 'Ebola virus VP40 drives the formation of virus-like filamentous particles along with GP' JOURNAL OF VIROLOGY vol. 76, no. 12, May 2002, pages 4855 - 4865, XP002976165
- TIMMINS J. ET AL.: 'Oligomerization and polymerization of the filovirus matrix protein VP40' VIROLOGY vol. 312, August 2003, pages 359 - 368, XP004446045
- RUIGROK R.W.H. ET AL.: 'Structural characterization and membrane binding properties of the matrix protein VP40 of ebola virus' J. MOL. BIOL. vol. 300, June 2000, pages 103 - 112, XP004486207
- YASUDA J. ET AL.: 'Nedd4 regulates egress of ebola virus-like particles from host cells' JOURNAL OF VIROLOGY vol. 77, no. 18, September 2003, pages 9987 - 9992, XP002976166
- KOLESNIKOVA L. ET AL.: 'VP40, the matrix protein of marburg virus, is associated with membranes of the late endosomal compartment' JOURNAL OF VIROLOGY vol. 76, no. 4, February 2002, pages 1825 - 1838, XP002976167

## Description

### INTRODUCTION

The filoviruses Ebola (EBOV) and Marburg (MBGV) are two of the most pathogenic viruses in humans and non-human primates (Feldman and Klenk, 1996, Adv. Virus Res. 47, 1), which cause a severe hemorrhagic fever (Johnson et al., 1997, Lancet 1, no. 8011, P. 569). The mortality rates associated with infections of Ebola or Marburg virus are up to 70-80% (Feldman and Klenk, 1996, supra; Johnson et al., 1997, supra). Although natural outbreaks have been geographically restricted so far, limited knowledge of the mechanisms of pathogenicity, potential of aerosol transmission (Jaax et al., 1995, Lancet 346, no. 8991-8992, 1669), unknown natural reservoir, and lack of immunological and pharmacological therapeutic measures, pose a challenge to classification of the public health threat of Marburg and Ebola viruses. Despite recent advances in vaccine development in certain animal models (Sullivan et al., 2000, Nature 408, 605; Vanderzanden et al., 1998, Virology 246, 134; Hevey et al., 1998, Virology 251, 28; Hevey et al., 1997, Virology 239, 206), substantial obstacles need to be overcome before such vaccines could qualify for human clinical trials (Burton and Parren, 2000, Nature 408, 569). Efforts to develop therapeutics against Ebola and Marburg have been hampered, in part, by limited knowledge of the mechanism of action of viral proteins and the poor understanding of the process of filovirus entry and budding at the molecular level.

WO00/00617 discloses Ebola virion proteins expressed from Venezuelan equine encephalitis (VEE) virus replicons. Harty et al, PNAS 97, 13871-13876 (2000) disclose a PPxY motif within the VP40 protein of Ebola virus that interacts physically and functionally with a ubiquitin ligase and its implications for filovirus budding.

Understanding the nature of interactions between filoviruses and the host, both at the cellular and organism levels, is essential for successful development of efficacious prophylactic and therapeutic measures.

Both entry and release of enveloped virus particles are dependent on an intimate interaction with components of the cellular membranes. While the plasma membrane was initially envisioned as a fluid, randomly arranged lipid bilayer with incorporated proteins, recent advances demonstrate that this important cellular barrier is more sophisticated and dynamic than portrayed by the original simplistic models. Cholesterol-enriched regions in the lipid bilayer have been recently defined that adopt a physical state referred to as liquid-ordered phase (lₒ) displaying reduced fluidity and the ability for lateral and rotational mobility (Simons and Ikonen, 1997, Nature 387, 569; Brown and London 1998, Annu. Rev. Cell Dev. Biol. 14, 111). These low density, detergent-insoluble microdomains, known as lipid rafts, accommodate a selective set of molecules such as gangliosides, glycosphingolipids, glycosylphosphatidylinositol (GPI) anchored proteins, and signaling proteins such as Src family kinases, T and B cell receptors, and phospholipase C (Simons and Ikonen, 1997, supra; Brown and London 2000, J. Biol. Chem 275, 17221; Simons and Toomre, 2000, Nature Rev. 1, 31; Aman and Ravichandran, 2000, Cur. Biol. 10, 393, Xavier et al., 1998, Immunity 8, 723). By virtue of these unique biochemical and physical properties, lipid rafts function as specialized membrane compartments for channeling certain external stimuli into specific downstream pathways (Cheng et al., 2001, Semin. Immunol. 13, 107; Janes et al., 2000, Semin. Immunol. 12, 23), act as platforms in cell-cell interactions (Viola et al., 1999, Science 283, 680; Moran and Miceli, 1998, Immunity 9, 787), and have also been implicated in membrane trafficking Brown and London, 1998, supra; Verkade and Simons, 1997, Histochem. Cell Biol. 108, 211). Lipid rafts are believed to perform such diverse functions by providing a specialized microenvironment in which the relevant molecules for the initiation of the specific biological processes are partitioned and concentrated (Brown and London, 2000, supra). Such compartmentalization may help the signals achieve the required threshold at the physiological concentrations of the stimuli. Partitioning in lipid rafts can also be perceived as a measure to perform functions in a more specific and economic manner while keeping distinct pathways spatially separated.

Several lines of evidence suggest a role for cholesterol-enriched lipid rafts in host-pathogen interactions. Cholesterol has been shown to play a critical role for the entry of mycobacterium into macrophages (Gatfield and pieters, 2000, Science 288, 1647). Multiple components of influenza virus (Scheiffele et al., 1999, J. Biol. chem. 274, 2038), measles virus (Manie et al., 2000, J. Virol. 74, 305), and human immunodeficiency virus (HIV) (Nguyen and Hildreth, 2000, J. Virol. 74, 3264; (Rousso et al., 2000, Proc. Natl. Acad. Sci. U.S.A. 97, 13523) have been shown to localize to lipid rafts. These lipid platforms have also been implicated in the budding of HIV and influenza virus (Scheiffele et al, 1999, supra; Nguyen and Hildreth, 2000, supra). Therefore, rafts, as tightly regulated specialized domains, may represent a coordination site for the intimate interactions of viral proteins required for the assembly and budding process. While involvement of rafts in virus entry has been postulated (Dimitrov, D. S. 2000, Cell 101, 687), supporting data on this issue have been reported only for HIV infection of certain T cell lines (Manes et al., 2000, EMBO Rep. 1, 190).

Therefore, there exists a need in the art for elucidation of the factors that affect filovirus assembly and disassembly. There is also a need for an efficient in vitro method for generation of genome-free virus-like particles which are stable, and retain immunogenic properties, i.e., those which present conformational, and more particularly, neutralizing epitopes expressed on the surface of native, intact filovirus.

Further, there is a need for elucidating the method by which filovirus enters a cell and exits a cell. Once the method is known, treatments and agents for disrupting attachment, fusion or entry of the virus, i.e. infection, can be ascertained.

### SUMMARY OF THE INVENTION

The present invention satisfies the needs discussed above. Using a variety of biochemical and microscopic approaches, we demonstrate the compartmentalization of Ebola and Marburg viral proteins in lipid rafts during viral assembly and budding. Our findings also show that filovirus trafficking, i.e. the entry and exit of filoviruses into and out of cells, is dependent on functional rafts. This study, thus, provides a deeper understanding of the molecular mechanisms of filovirus pathogenicity at the cellular level, and suggests raft integrity and/or raft components as potential targets for therapeutic interventions. We also report, for the first time, the raft- dependent formation of Ebola-based and Marburg-based, genome-free, virus-like particles (VLPs), which resemble live virus in electron micrographs. Such VLPs, besides being a research tool, could be potentially useful as vaccines against filovirus infections, or as vehicles for the delivery to cells of a variety of antigens artificially targeted to the rafts.

Therefore, the present invention relates to filovirus virus-like particles (VLPs) comprising filovirus envelope glycoprotein GP, and filovirus matrix protein, VP40. Also described herein is a method for generating genome-free Ebola or Marburg VLPs in a mammalian transfection system. This method generates VLPs that resemble native virus. The virus-like particles are useful for transferring into a cell a desired antigen or nucleic acid which would be contained in the internal space provided by the virus-like particles.

The invention further provides a method of producing a filovirus virus like particle (VLP) comprising expressing in a cell a polynucleotide encoding filovirus envelope glycoprotein, GP, and filovirus matrix protein, VP40 such that said polynucleotide is expressed and said VLP is produced. In related embodiments, the invention provides an Ebola virus like particle (VLP) producing cell expressing Ebola GP and VP40 and a Marburg virus like particle (VLP) producing cell expressing Marburg GP and VP40. Additionally described herein is a method for generating genome-free filovirus virus-like particles (VLPs), specifically, Ebola and Marburg VLPs. The method includes expression of virus GP and VP40 in cells. The VLP of the present invention are more native in the filovirus-like morphology and more native in terms of the conformation of virus spikes.

Also described herein are VLP-containing compositions. The compositions contain Ebola VLPs or Marburg VLPs or a combination of Ebola and Marburg VLPs for use as a vaccine, a delivery vehicle and in a diagnostic assay. Further described herein is a filovirus vaccine comprising a virus like particle (VLP) comprising filovirus envelope glycoprotein, GP, and filovirus matrix protein, VP40, or a filovirus vaccine comprising a VLP obtainable by a method of the invention. Additionally described herein is a filovirus vaccine comprising a virus like particle (VLP) and a nucleic acid encoding an agent capable of eliciting an immune response against said filovirus.

The invention additionally provides an immunogenic composition comprising, in a physiologically acceptable vehicle, Ebola virus like particles (VLPs) or Marburg virus like particles (VLPs).

Further described herein is a vaccine for inducing an immune response to a filovirus, namely Ebola or Marburg, said vaccine comprising Ebola VLP or Marburg VLP, respectively, or a combination of Ebola and Marburg VLPs.

The invention provides Ebola virus like particles (VLPs) or Marburg virus like particles (VLPs) for use in a method of stimulating an Ebola or Marburg virus specific immune response. The invention further provides a use of Ebola virus like particles (VLPs) or Marburg virus like particles (VLPs) in the manufacture of a medicament for stimulating an Ebola or Marburg virus specific immune response.

There is also described herein a method for encapsulating desired agents into filovirus VLP, e.g., therapeutic or diagnostic agents.

Further described herein are filovirus VLPs, preferably Ebola VLPs or Marburg VLPs, which contain desired therapeutic or diagnostic agents contained therein, e.g. anti-cancer agents or antiviral agents.

Additionally described herein is a novel method for delivering a desired moiety, e.g. a nucleic acid to desired cells wherein the delivery vehicle for such moiety comprises filovirus VLP. In this aspect, the disclosure provides a method for introducing an agent into a cell, comprising packaging said agent into a virus like particle (VLP), producing a packed VLP and allowing the packed VLP to enter said cell, wherein the VLP comprises filovirus envelope glycoprotein, GP, and filovirus matrix protein, VP40, or is a VLP obtainable by a method of the invention.

A diagnostic assay for the detection of Ebola or Marburg virus infection in a sample from a subject suspected of having such an infection is described herein. The method comprises detecting the presence or absence of a complex formed between anti-Ebola antibodies or antiMarburg antibodies in the sample and Ebola VLPs or Marburg VLPs, respectively.

In a related aspect, the invention provides a method for detecting Ebola virus infection or Marburg virus infection comprising contacting a sample from the subject suspected of having Ebola virus infection or Marburg virus infection with an Ebola or Marbug virus like particle (VLP) of the invention and detecting the presence or absence of an infection by detecting the presence or absence of a complex formed between the VLP and antibodies specific therefor in said sample. In another related aspect, the invention provides a kit for the detection of Ebola virus or Marburg virus infection comprising Ebola or Marburg virus like particles (VLPs) of the invention.

Further described herein is the use of noninfectious filovirus VLP in an in vitro assay for testing the efficacy of potential agents to inhibit or reduce filovirus entry into cells or budding from cells, i.e. infectivity. In this aspect the invention provides a method for testing an agent involved in filovirus budding, comprising introducing said agent to a cultured cell producing filovirus virus like particle (VLP) and monitoring the presence or absence of a change in the budding of VLP as compared to a control by measuring VLPs in supernatant of said cultured cell, wherein a reduction or increase in the number of VLP in the supernatant indicates a negative or positive agent, respectively, on filovirus budding.

Additionally described herein is a method for identifying critical structural elements within filovirus proteins required for viral assembly and/or release. The method consists of detecting a change in VLP formation, assembly, or budding from a cell expressing filovirus mutant proteins as compared to a cell expressing wild type alleles of such mutations.

Also described herein is an immunological composition for the protection of mammals against Ebola or Marburg virus infection comprising Ebola or Marburg virus-like particles.

Further described herein is a method for evaluating effectiveness of an agent or chemical to block entry of filovirus into a cell, said agent or chemical able to alter the cell's lipid rafts, said method comprising introducing said agent or chemical to a cell and monitoring the effect of said agent or chemical by monitoring VLP entry or exit from a cell. Agents include chemicals, cellular agents or factors, and other viral agents.

### BRIEF DESCRIPTION OF THE DRAWINGS

These and other features, aspects, and advantages of the present invention will become better understood with reference to the following description and appended claims, and accompanying drawings where:

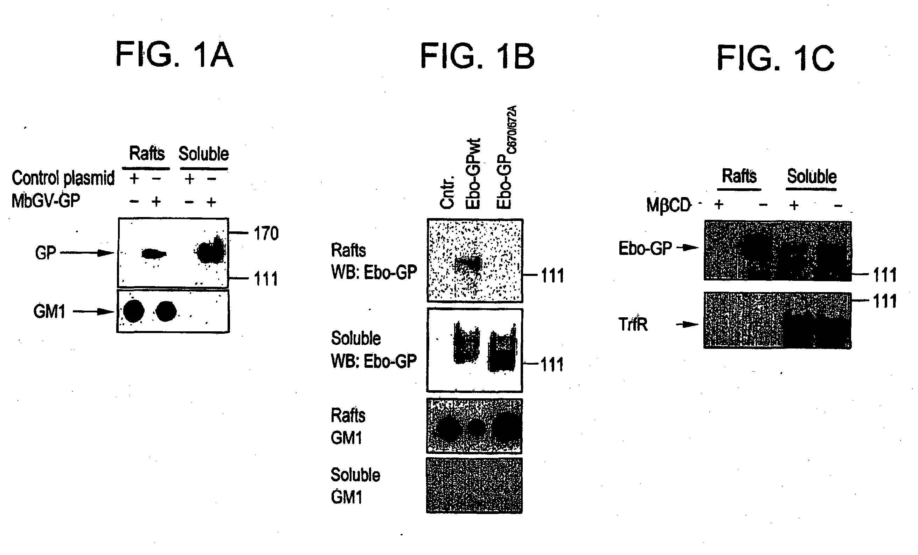

**Figure 1A, 1B, and 1C****.** *Localization of filovirus glycoproteins in lipid rafts.* 293T cells were transfected with Marburg GP (**A**), Ebo-GPwt, or Ebo-GP_{C670/672A} (**B**), or a control plasmid, rafts were prepared by ultracentrifugation and GP was detected by immunoblotting. GM1 was detected by blotting with HRP-CTB in the corresponding fractions spotted on a nitrocellulose membrane, as a control for the quality of raft preparation. (**C**) 48h after transfection of 293T cells with Ebola GP, a portion of cells were treated for 20 minutes with 10 mM methyl-b-cyclodextrin (MbCD) and another portion was left untreated. Raft and soluble fractions were prepared and analyzed by immunoblotting for GP (upper panel) and for the raft-excluded protein transferrin receptor (TrfR, lower panel).

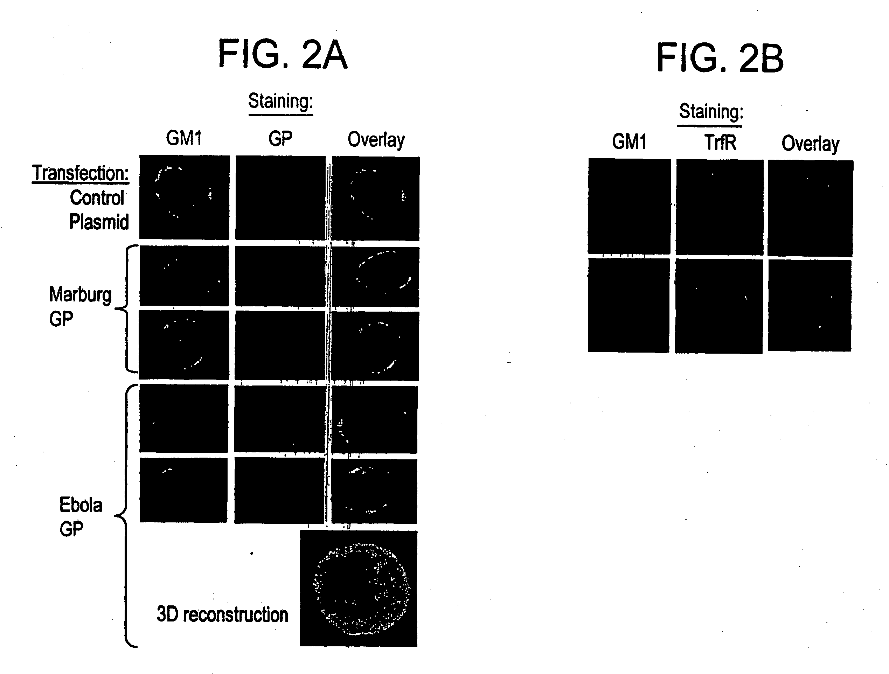

**Figure 2A and 2B****.** *Colocalization of filovirus, glycoproteins with GM1 on intact cells.* (**A**) 293T cells were transected with the indicated GP, and stained at 4°C with Alexa488-CTB (green) and anti-GP mAb followed by Alexa-647 conjugated anti-mouse antibodies (red), cells were fixed and imaged using confocal microscopy. Colocalization is represented by yellow appearance in the overlay (right panels). A 3-D reconstruction of the compiled data from 25 sections of a Ebo-GP transfected cell is also shown. (*B*) 293T cells were concurrently stained at 4°C with Alexa-488 conjugated anti-TrfR antibody (green) and Rohdamin-CTB (red), fixed and analyzed by confocal microscopy. No colocalization between these two molecules was observed, evident by the lack of yellow appearance.

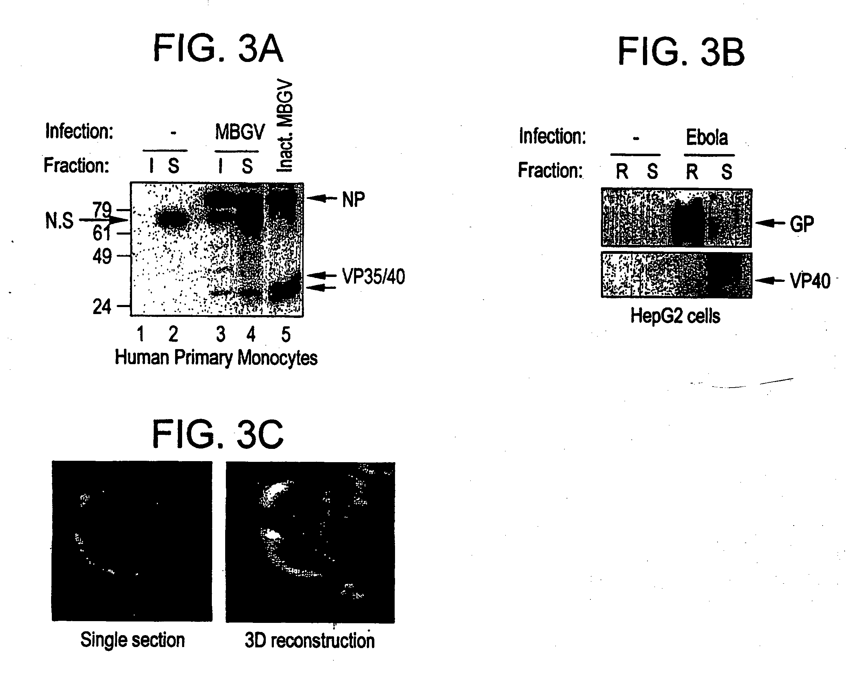

**Figure 3A, 3B and 3C****.** *Localization of filovirus proteins in lipid rafts in infected cells.* **A**. Primary human monocytes were infected with MBGV. After 24h cells where lysed in 0.5% triton-X100 and detergent-soluble (S) and -insoluble (I) fractions were separated by centrifugation, samples were irradiated (2x10⁶ R), and analyzed by immunoblotting with a guinea pig anti-MBGV antibody to detect viral proteins NP and VP35/VP40 (lanes 3,4); lanes 1,2: uninfected control; lane 5: inactivated MBGV (1 mg). N.S.: non-specific band. ***B***. HepG2 hepatocytes were infected with EBOV-Zaire, lysed, irradiated (6x10⁶ R), and rafts (R) and soluble (S) fractions where prepared by ultracentrifugation 24 hours post infection. Ebola GP and VP40 were detected by immunoblotting. ***C***. Ebola-infected Vero E6 cells were irradiated (4x10⁶ R), fixed and stained for Ebola virus (red) and GM1 (green) at 4°C and imaged by confocal microscopy; left panel: single section; right panel: 3D reconstruction of the compiled data.

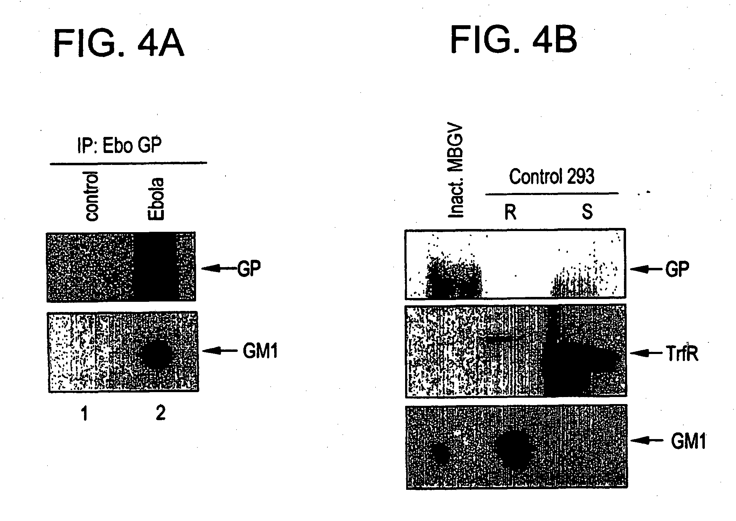

**Figure 4A and 4B****.** *Incorporation of GM1 in released filovirus virions.* (**A**). Ebola virus was immunoprecipitated from supernatant of infected Vero-E6 cells (lane 2), or uninfected cells as control (lane 1), using anti-GP mAb. After irradiation (2x10⁶ R), a fraction of immunoprecipitate (IP) was spotted on nitrocellulose membrane and blotted with HRP-conjugated CTB to detect GM1 (lower panel). Another portion of the IP was analyzed by SDS-PAGE and immunoblotting with anti-GP mAb (top panel). (*B*) MBGV (1 mg), prepared by ultracentrifugation and inactivated by radiation (1x10⁷ R), was analyzed for the presence of GM1, TrfR and GP in a similar fashion. As control, rafts and soluble fractions from untransfected 293T cells were used.

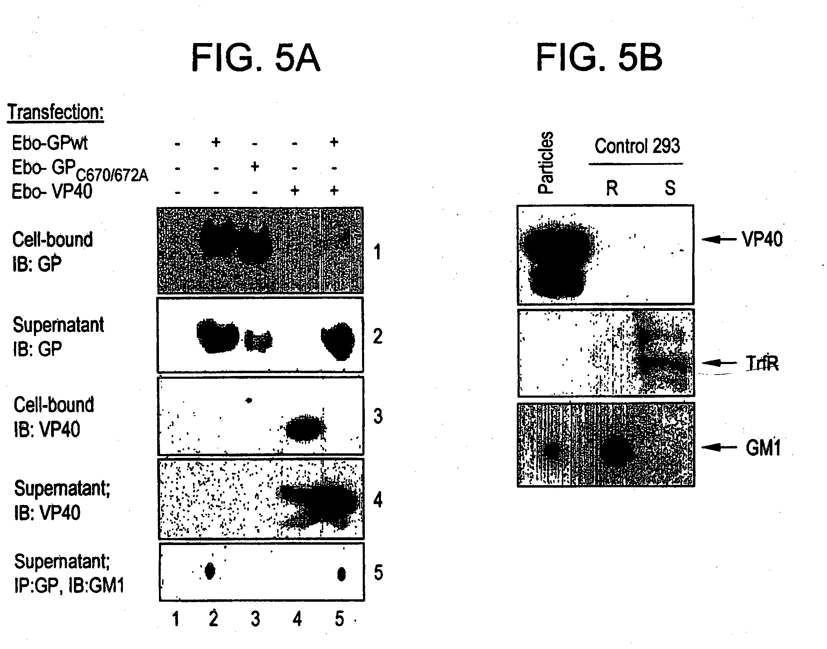

**Figure 5A and 5B****.** *Release of Ebola GP and VP40 as GM1-containing particles.* (***A***) 293T cells were transfected with the indicated plasmids, supernatants were cleared from floating cells by centrifugation and particulate material were pelleted through 30% sucrose by ultracentrifugation. The individual proteins were detected in the cell lysates and in the particulate material from supernatant by immunoblotting (IB). A fraction of cleared supernatant was subjected to
   immunoprecipitation using anti-GP mAb and analyzed for the presence of GM1 (lower panel) as described in the legend to Figure 1. (**B**) The particulate material from cells transfected with GP+VP40 were further purified on a sucrose step gradient and the low density fraction was analyzed for the presence of VP 40 (top panel), TrfR (middle panel), and GM1 (lower panel). Rafts and soluble fractions from untransfected 293T cells were used as control.

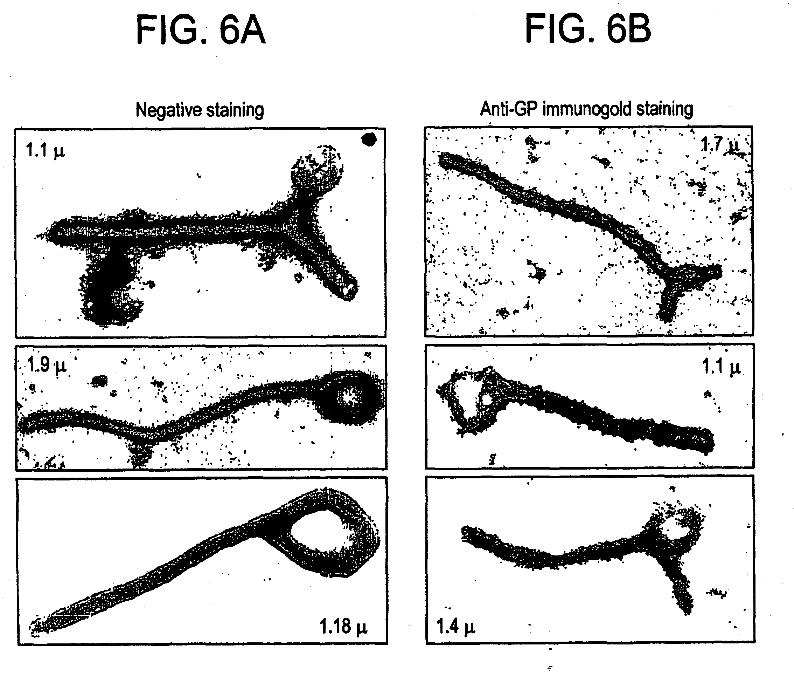

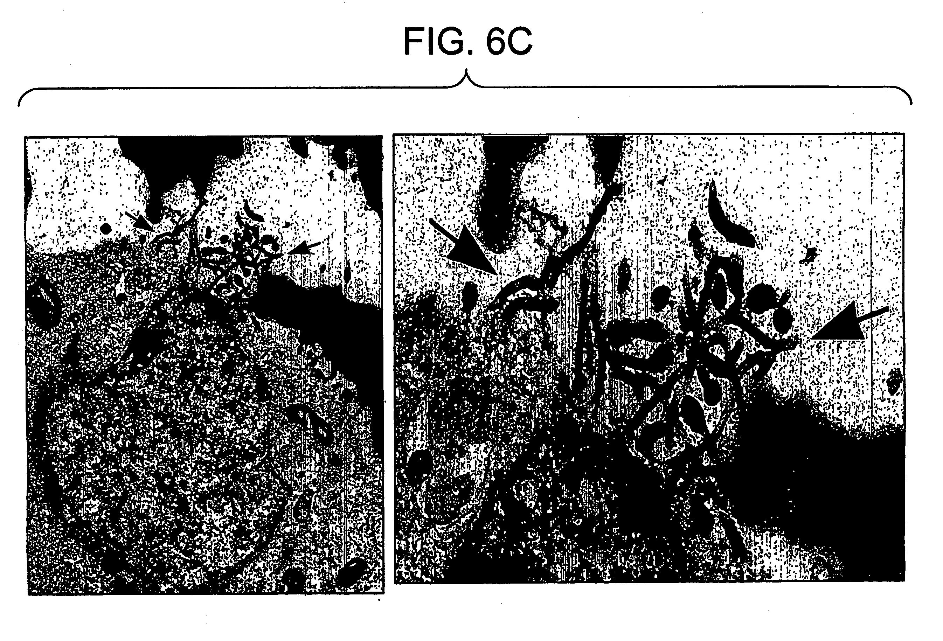

**Figure 6A, 6B****, and** **6C****.** *Electron microscopic analysis of virus like particles generated by EBOV GP and VP40. Particles* obtained by ultracentrifugation of the supernatants of 293T cells transfected with Ebola GP+VP40 were negatively stained with uranylacetate to reveal the ultrastructure (***A***), or stained with anti-Ebo-GP mAb followed by Immunogold rabbit anti mouse Ab (***B***), and analyzed by electron microscopy. The length of each particle is indicated in mm. (***C***) 293T cells transfected with EbolaGP+VP40 were immunogold-stained for Ebola GP, fixed, cut, and analyzed by electron microscopy. The picture depicts a typical site of VLP release from the cells, indicated by arrows. A magnification of the site of VLP release is also shown to better visualize the gold staining on the particles.

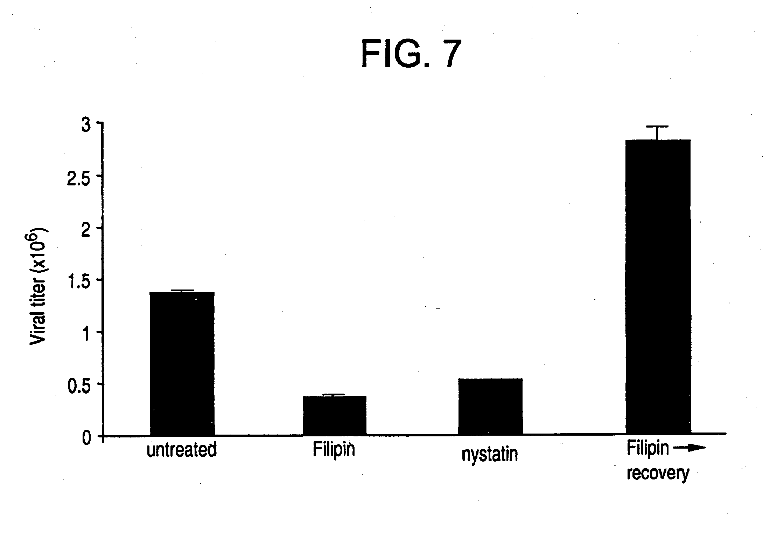

**Figure 7****.** *Inhibition of Ebola infection by raft-disrupting agents filipin and nystatin:* Vero E6 cells were left untreated or treated for 30 minutes with 0.2 mg/ml of filipin or 100U/ml of nystatin at 37°C, washed extensively with PBS and infected with Ebola at an MOI of 1. As a control for lack of general toxicity and persistent effect on viral replication, upon treatment with filipin, cells were washed and incubated in medium for 4h before infection with EBOV (Filipin(recovered). After 48h supernatants were harvested and viral titers determined by plaque assay.

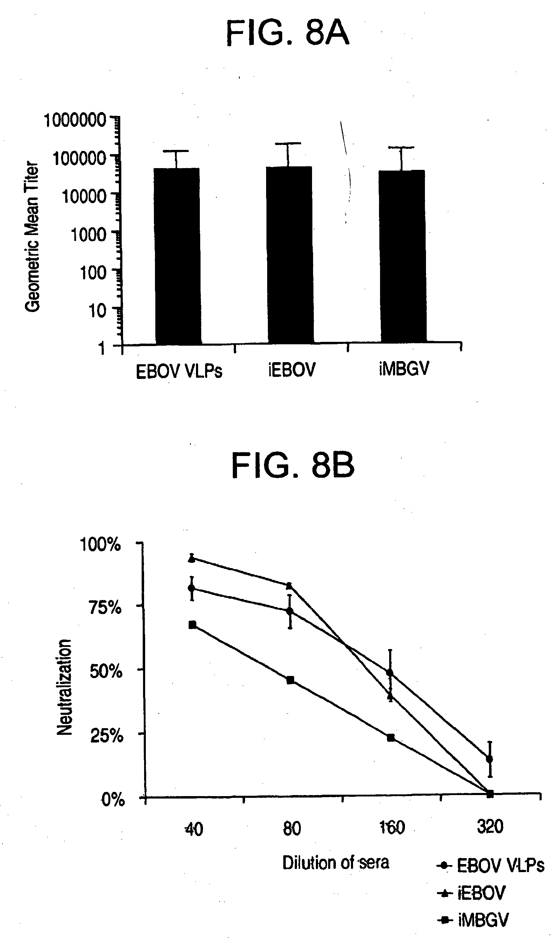

**Figure 8A and 8B****.** Serum antibody responses in mice following intraperitoneal immunization with 40 ug of EBOV VLPs, inactivated Ebola (iEBOV) or Marburg (iMBGV) virus on days 0, 21, and 42. (A) Total IgG serum anti-Ebola antibodies were measured by ELISA 42 and 63 days post immunization (dpi) following the 2nd or 3rd vaccination, respectively. Ebola antibody titers were measured for individual mice and the results are graphed as the endpoint titer for each mouse. The number of mice with the same endpoint titer are noted on the graph. Closed and filled symbols represent the titer after second and third vaccination respectively. (B) Percent neutralization of Ebola virus infection in VeroE6 cells by sera of immunized mice. Two-fold dilutions of sera were tested for their ability to neutralize Ebola virus infection and are plotted as the mean of the percent neutralization for each group ,of immune sera as compared to mock-treated VeroE6 cells.

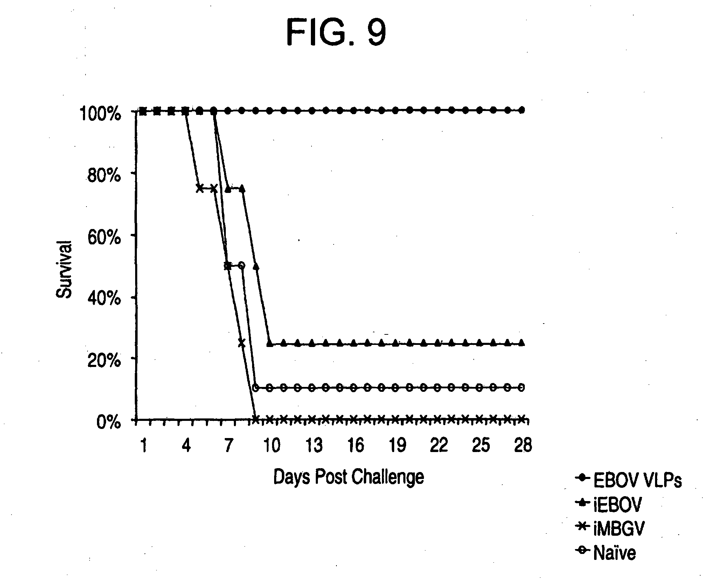

**Figure 9****.** EBOV VLPs protect mice against challenge with mouse-adapted EBOV. Mice were immunized intraperitoneally with 40 ug of EBOV VLPs, iEBOV or iMBGV on 0, 21, and 42 dpi. All mice were challenged on day 63 with 300 pfu of mouse-adapted Ebola virus. Results are plotted as percent survival for each immunization group.

### DETAILED DESCRIPTION

In the description that follows, a number of terms used in recombinant DNA, Virology and immunology are extensively utilized. In order to provide a clearer and consistent understanding of the specification and claims, including the scope to be given such terms, the following definition are provided.

**Filoviruses.** The filoviruses (e.g. Ebola virus (EBOV) and Marburg virus (MBGV)) cause acute hemorrhagic fever characterized by high mortality. Humans can contract filoviruses by infection in endemic regions, by contact with imported primates, and by performing scientific research with the virus. However, there currently are no available vaccines or effective therapeutic treatments for filovirus infection. The virions of filoviruses contain seven proteins which include a surface glycoprotein (GP), a nucleoprotein (NP), an RNA-dependent RNA polymerase (L), and four virion structural proteins (VP24, VP30, VP35, and VP40).

**Subject.** Includes human, animal, avian, e.g., horse, donkey, pig, mouse, hamster, monkey, chicken, and insect such as mosquito.

**Virus-like particles (VLP).** This refers to a structure which resembles the outer envelope of the native virus antigenically and morphologically. The virus-like particles are formed in vitro upon expression, in a cell, of viral surface glycoprotein (GP) and a virion structural protein, VP40. It may be possible to produce VLPs by expressing only portions of GP and VLP40.

The present invention generally relates to a novel method for producing VLP from filovirus , e.g., Ebola and Marburg virus. The method expresses in a cell a polynucleotide encoding viral glycoprotein GP and the virion structural protein, VP40 such that the polynucleotide is expressed and VLP is produced. Expression of GP and VP40 may be achieved by transfection of DNA fragments which encode these proteins into the desired cells. DNA fragments which encode any of the Ebola Zaire 1976 or 1995 (Mayinga isolate) GP and VP40 proteins are described herein. Accession# AY142960 contains the whole genome of Ebola Zaire, with individual genes including GP and VP40 specified in this entry, VP40 gene nucleotides 4479-5459, GP gene 6039-8068. The entire Marburg genome has been deposited in accession # NC_001608 for the entire genome, with individual genes specified in the entry, VP40 gene 4567-5478, GP gene 5940-7985, NP gene 103-2190. The protein ID for Ebola VP40 is AAN37506.1, for Ebola GP is AAN37507.1, for Marburg VP40 is CAA78116.1, and for Marburg GP is CAA78117.1. The DNA fragments were inserted into a mammalian expression vector, specifically, pWRG7077, and transfected into cells.

Also described herein is a recombinant DNA molecule that includes a vector and a DNA sequence as described above. The vector can take the form of a plasmid, a eukaryotic expression vector such as pcDNA3.1, pRcCMV2, pZeoSV2, or pCDM8, which are available from Invitrogen, or a virus vector such as baculovirus vectors, retrovirus vectors or adenovirus vectors, alphavirus vectors, and others known in the art. The minmum requirement is a promoter that is functional in mammalian cells for expressing the gene.

A suitable construct for use in the method of the present invention is pWRG7077 (4326 bp)(PowderJect Vaccines, Inc., Madison, WI). pWRG7077 includes a human cytomegalovirus (hCMV) immediate early promoter and a bovine growth hormone polyA addition site. Between the promoter and the polyA addition site is Intron A, a sequence that naturally occurs in conjunction with the hCMV IE promoter that has been demonstrated to increase transcription when present on an expression plasmid. Downstream from Intron A, and between Intron A and the polyA addition sequence, are unique cloning sites into which the desired DNA can be cloned. Also provided on pWRG7077 is a gene that confers bacterial host-cell resistance to kanamycin. Any of the fragments that encode Ebola GP, Ebola VP40, Marburg GP, and Marburg VP40 can be cloned into one of the cloning sites in pWRG7077, using methods known to the art.

All filoviruses have GP proteins that have similar structure, but with allelic variation. By allelic variation is meant a natural or synthetic change in one or more amino acids which occurs between different serotypes or strains of Ebola or Marburg virus and does not affect the antigenic properties of the protein. There are different strains of Ebola (Zaire 1976, Zaire 1995, Reston, Sudan, and Ivory Coast with 1-6 species under each strain). Marburg has species Musoke, Ravn, Ozolin, Popp, Ratayczak, Voege. The GP and VP genes of these different viruses have not been sequenced. It would be expected that these proteins would have homology among different strains. It is reasonable to expect that similar VLPs from other filoviruses can be prepared by using the concept of the present invention described for MBGV and EBOV, i.e. expression of GP and VP40 genes from other filovirus strains would result in VLPs specific for those strains.

Host cells stably transformed or transfected with the above-described recomhinant DNA constructs or expressing said DNA are described herein. The host cell can be prokaryotic (for example, bacterial), lower eukaryotic (for example, yeast or insect) or higher eukaryotic (for example, all mammals, including but not limited to mouse and human). Both prokaryotic and eukaryotic host cells may be used for expression of the desired coding sequences when appropriate control sequences which are compatible with the designated host are used. Host cells include all cells susceptible to infection by filovirus.

Among prokaryotic hosts, *E*. *coli* is the most frequently used host cell for expression. General control sequences for prokaryotes include promoters and ribosome binding sites. Transfer vectors compatible with prokaryotic hosts are commonly derived from a plasmid containing genes conferring ampicillin and tetracycline resistance (for example, pBR322) or from the various pUC vectors, which also contain sequences conferring antibiotic resistance. These antibiotic resistance genes may be used to obtain successful transformants by selection on medium containing the appropriate antibiotics. Please see e.g., Maniatis, Fitsch and Sambrook, Molecular Cloning; A Laboratory Manual (1982) or DNA Cloning, Volumes I and II (D. N. Glover ed. 1985) for general cloning methods.

In addition, the filovirus gene products can also be expressed in eukaryotic host cells such as yeast cells and mammalian cells. *Saccharomyces cerevisiae, Saccharomyces carlsbergensis,* and *Pichia pastoris* are the most commonly used yeast hosts. Control sequences for yeast vectors are known in the art. Mammalian cell lines available as hosts for expression of cloned genes are known in the art and include many immortalized cell lines available from the American Type Culture Collation (ATCC), such as HEPG-2, CHO cells, Vero cells, baby hamster kidney (BHK) cells and COS cells, to name a few. Suitable promoters are also known in the art and include viral promoters such as that from SV40, Rous sarcoma virus (RSV), adenovirus (ADV), bovine papilloma virus (BPV), and cytomegalovirus (CMV). Mammalian cells may also require terminator sequences, poly A addition sequences, enhancer sequences which increase expression, or sequences which cause amplification of the gene. These sequences are known in the art.

The transformed or transfected host cells can be used as a source of DNA sequences described above. When the recombinant molecule takes the form of an expression system, the transformed or transfected cells can be used as a source of the VLP described below.

Cells may be transfected with one or more expression vector expressing filovirus GP and VP40 using any method known in the art, for example, calcium phosphate transfection as described in the examples. Any other method of introducing the DNA such that the encoded proteins are properly expressed can be used, such as viral infections, electroporation, to name a few.

For preparation of VLPs, supernatants are collected from the above-described transfected cells, preferably 60 hours post-transfection. Other times can be used depending on the desired number of intact VLPs. Our endpoint is the greatest number of intact VLPs, we could use other times which will depend on how we express the genes. Presumably an inducible system would not require the same length of incubation as transient transfection. The supernatants then separated on a 20% gradient in order to concentrate the VLPs and reduce contamination from cellular material. The partially purified material is then separated on a 30% sucrose gradient. The isolation technique will depend upon factors such as the specific host cells used, concentration, whether VLPs remains intracellular or are secreted, among other factors. The isolated VLPs are about 95% pure with a low enough endotoxin content for use as a vaccine. In these instances, the VLP used will preferbly be at least 10-30% by weight, more preferably 50% by weight, and most preferably at least 70-90% by weight. Methods of determining VLP purity are well known and include SDS-PAGE densitometric methods.

The resulting VLPs are not homogeneous in size and exhibit conformational, neutralizing epitopes found on the surface of authentic Ebola or Marburg virions. The VLPs are comprised of GP and VP40. Other proteins can be added such as NP, VP24, and VP35 without affecting the structure.

While these results are novel and unexpected, based on the teachings of this application, one skilled in the art may achieve greater VLP yields by varying conditions of transfection and separation.

A single-component vaccine protective against filovirus is described herein. VLPs should be recognized by the body as immunogens but will be unable to replicate in the host due to the lack of appropriate viral genes, thus, they are promising as vaccine candidates. The filoviruses may be MBGV or EBOV. A vaccine described herein may comprise one or more VLP derived from cells espressing EBOV GP and VP40 for use as an Ebola vaccine, or VLP derived from cells expressing or MBGV GP and VP40 for use as a Marburg vaccine. Even though the specific strains of EBOV and MBGV were used in the examples below, it is expected that protection would be afforded using VLPs from other MBGV strains and isolates, and/or other EBOV strains and isolates.

Additionally described herein is a method for providing immunity against MBGV and EBOV virus said method comprising administering one or more VLP to a subject such that a protective immune reaction is generated.

Vaccine formulations described herein comprise an immunogenic amount of VLPs or a combination of VLPs as a multivalent vaccine, in combination with a pharmaceutically acceptable carrier. An "immunogenic amount" is an amount of the VLPs sufficient to evoke an immune response in the subject to which the vaccine is administered. An amount of from about 10⁵ to 10⁸ or more VLPs per dose with one to three doses one month apart is suitable, depending upon the age and species of the subject being treated. Exemplary pharmaceutically acceptable carriers include, but are not limited to, sterile pyrogen-free water and sterile pyrogen-free physiological saline solution.

Administration of the VLPs disclosed herein may be carried out by any suitable means, including both parenteral injection (such as intraperitoneal, subcutaneous, or intramuscular injection), by in ovo injection in birds, orally and by topical application of the VLPs (typically carried in the pharmaceutical formulation) to an airway surface. Topical application of the VLPs to an airway surface can be carried out by intranasal administration (e.g. by use of dropper, swab, or inhaler which deposits a pharmaceutical formulation intranasally). Topical application of the VLPs to an airway surface can also be carried out by inhalation administration, such as by creating respirable particles of a pharmaceutical formulation (including both solid particles and liquid particles) containing the VLPs as an aerosol suspension, and then causing the subject to inhale the respirable particles. Methods and apparatus for administering respirable particles of pharmaceutical formulations are well known, and any conventional technique can be employed.

The vaccine may be given in a single dose schedule, or preferably a multiple dose schedule in which a primary course of vaccination may be with 1-10 separate doses, followed by other doses given at subsequent time intervals required to maintain and or reinforce the immune response, for example, at 1-4 months for a second dose, and if needed, a subsequent dose(s) after several months. Examples of suitable immunization schedules include: (i) 0, 1 months and 6 months, (ii) 0, 7 days and 1 month, (iii) 0 and 1 month, (iv) 0 and 6 months, or other schedules sufficient to elicit the desired immune responses expected to confer protective immunity, or reduce disease symptoms, or reduce severity of disease.

A method of detecting the presence of antibodies against Ebola virus or Marburg virus in a sample is described herein. Using standard methodology well known in the art, a diagnostic assay can be constructed by coating on a surface (i.e. a solid support for example, a microtitration plate, a membrane (e.g. nitrocellulose membrane) or a dipstick, all or a unique portion of any of the Ebola or Marburg VLPs described above, and contacting it with the serum of a person or animal suspected of having an infection. The presence of a resulting complex formed between the VLPs and serum antibodies specific therefor can be detected by any of the known methods common in the art, such as fluorescent antibody spectroscopy or colorimetry. This method of detection can be used, for example, for the diagnosis of Ebola or Marburg infection and for determining the degree to which an individual has developed virus-specific Abs after administration of a vaccine.

A diagnostic kit is further described herein which contains the VLPs described above and ancillary reagents that are well known in the art and that are suitable for use in detecting the presence of antibodies to Ebola or Marburg in serum or a tissue sample. Tissue samples contemplated can be from monkeys, humans, or other mammals. Additionally described herein is a method for producing VLPs which have encapsulated therein a desired moiety.

The moieties that may be encapsulated in the VLP include therapeutic and diagnostic moieties, e.g., nucleic acid sequences, radionuclides, hormones, peptides, antiviral agents, antitumor agents, cell growth modulating agents, cell growth inhibitors, cytokines, antigens, toxins, etc. The moiety encapsulated should not adversely affect the VLP, or VLP stability. This may be determined by producing VLP containing the desired moiety and assessing its effects, if any, on VLP stability.

The subject VLP, which contain a desired moiety, upon administration to a desired host, should be taken up by cells normally infected by the particular filovirus, e.g., epithelial cells, keratinocytes, etc. thereby providing for the potential internalization of said moiety into these cells. This may facilitate the use of subject VLPs for therapy because it enables the delivery of a therapeutic agent(s) into a desired cell, site, e.g., a cervical cancer site. This may provide a highly selective means of delivering desired therapies to target cells.

In case of DNAs or RNAs, the encapsulated nucleic acid sequence can be up to 19 kilobases, the size of the particular filovirus. However, typically, the encapsulated sequences will be smaller, e.g., on the order of 1-2 kilobases. Typically, the nucleic acids will encode a desired polypeptide, e.g., therapeutic, such as an enzyme, hormone, growth factor, etc. This sequence will further be operably linked to sequences that facilitate the expression thereof in the targeted host cells.

Additionally described herein is a diagnostic assay for identifying agents which may cause disassembly of the VLP, or agents which can inhibit budding of virus from the host cell, or agents which inhibit filovirus entry into or exit from a cell. Such agents may include altered viral proteins, cellular factors, and chemical agents.

A diagnostic assay for agents which might inhibit viral budding comprises:
(i) contacting cells expressing VP40 and GP from a filovirus and producing VLPs with an agent thought to prevent viral budding from cells; and
(ii) monitoring the ability of said agent to inhibit VLP budding from cells by detecting an increase or decrease of VLPs in cell culture supernatant, wherein a decrease in VLPs in the supernatant indicates an inhibitory activity of said agent. This would include the generation of VLPs containing fluorescent tags attached to GP or VP40 to make the VLP generation trackable in high throughput screening assays.

A diagnostic assay for screening agents which inhibit viral entry into cells comprises:
(i) treating cells with an agent suspected of inhibiting viral entry;
(ii) contacting treated cells with filovirus VLPs;
(iii) detecting a change in the number of VLPs able to enter treated cells compared to untreated cells wherein a decrease in the number of VLPs in treated cells indicated an inhibitory activity of said agent. VLP entry into cells can be monitored using lipophilic dyes.

Further described herein is a diagnostic kit which contains cells expressing filovirus proteins GP and VP40 such that VLPs of said filovirus are produced and ancillary reagents suitable for use in detecting the presence of VLPs in the supernatant of said cells when cultured. Said cells would include any mammalian cell, for example, 293T, VERO, and other mammalian cells expressing VP40 and GP from Ebola virus or expressing VP40 and GP from Marburg virus.

Applicants for the first time have identified lipid rafts as a gateway for entry and exit from a cell. Stable lipid rafts serve as the site of filovirus assembly and budding. Therefore, a method is described herein for inhibiting entry of filovirus into cells, said method comprising inhibiting the association of the virus with lipid rafts in cells. Such methods would include providing a cell which produces filovirus VLP, administering a lipid rafts destabilizing agent, and monitoring the effect of the agent on filovirus entry by monitoring the amount of VLPs entering the cell as compared to a control of untreated cells, or alternatively, monitoring the effect of the agent on filovirus budding from the cell by monitoring the amount of VLPs in the culture supernatant as compared to a control of untreated cells.

Agents which destablitize lipid rafts include filipin, nystatin, and other cholesterol synthesis inhibitors known collectively as statins such as methyl-β-cyclodextrin, or agents which compete with the virus for binding to lipid rafts, such agents, including mutant VP40 or mutant GP, e.g. having mutations which inhibit palmitoylation at cystein residues 670 and 672.

The following examples are included to demonstrate preferred embodiments of the invention. It should be appreciated by those of skill in the art that the techniques disclosed in the examples which follow represent techniques discovered by the inventors and thought to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result.

### Materials and Methods:

*Plasmids, transfections, western blot, GM1 blot:* cDNAs encoding Ebola-Zaire GP and VP40 as well as MBGV Musoke GP were cloned in pWRG7077 mammalian expression vector. 293 T cells were transfected using calcium phosphate transfection kit (Edge Biosystems, Gaithersburg, Maryland) according to manufacturer's instructions. Western blot analysis was performed using as primary antibodies anit-EboGP mAb 13F6 (Wilson et al., 2000, Science 287, 1664), anti-Marburg GP mAb (5E2) (Dr. Michael Hevey, USAMRIID) anti Ebo-VP40 mAb (Dr. Connie Schmaljohn, USAMRIID) or a guinea pig anti-Marburg antibody (Dr. Michael Hevey, USAMRIID), followed by blotting with HRP-conjugated secondary antibodies and signals were detected by enhanced chemiluminescence. GM1 was detected in lysates or immunoprecipitates by spotting on a nitrocellulose membrane after boiling in SDS, followed by blocking of the membranes and blotting with HRP-conjugated CTB and detection by ECL.

*Preparation of detergent insoluble fractions and lipid rafts:* Lipid rafts were prepared after lysing the cells in lysis buffer containing 0.5% Triton-X100 as previously described (Aman and Ravishandran, 2000, supra). Raft and soluble fractions were then analyzed by immunoblotting. In some experiments (Figure 3A), detergent-insoluble fraction was extracted without ultracentrifugation as described previously (Rousso et al., 2000, supra). Briefly, cells were pelleted and lysed in 0.5% Triton-X100 lysis buffer. After removing the lysate (soluble fraction), the pellet was washed extensively and SDS sample bluffer added to pellet (insoluble fraction). Soluble and insoluble fractions were analyzed by SDS page and immunoblotting.

*Cell culture, infections, virus and VLP purification*: Peripheral blood mononuclear cells (PBMC) were isolated by density centrifugation through Ficoll-Paque (Amerhsam/Pharmacia, Piscataway, NJ) according to manufacturer's instructions. PBMCs were cultured in RPMI/10% fetal bovine serum for 1 hour at 37°C, 5% CO₂ after which non-adherent cells were removed. Adherent cells were cultured for an additional 5 days. HEPG2 cells (ATCC, Manassas, Virginia) were cultured to confluency with complete RPMI 1640 prior to use. Monocyte derived macrophages, HEPG2 cells, and Vero-E6 cells were infected at a multiplicity of infection (M.O.I.) of 1 with either Ebola-Zaire or Marburg Musoke virus for 50 minutes at 37°C, 5% CO₂. Non-adsorbed virus was removed from cells by washing monolayers twice with PBS followed by addition of fresh complete medium for an additional 24-48 hours. Purification and inactivation of Marburg virus was performed as previously described (Hevey et al., 1997, supra). Briefly, Vero-E6 cells were infected with MBGV and supernatant was harvested 6-7 days post-infection. The medium was clarified and virus concentrated by polyethylene glycol precipitation. After centrifugation at 10,000 g for 30 min, pellets were resuspended in Tris buffer and layered atop 20-60% sucrose gradients and centrifuged at 38,000 rpm for 4 hr. The visible virus band was collected. Samples were inactivated by irradiation (10⁷ R, ⁶⁰Co source) and tested for absence of infectivity in cell culture before use. For preparation of VLPs, supernatants were collected 60h post-transfection, overlaid on 30% sucrose and ultracentrifuged at 26000 rpm for 2 hours. Pelleted particulate material was recovered in PBS and analyzed by immunoblotting or electron microscopy. As a further purification step, in some experiments this particulate material was loaded on a step gradient consisting of 80%, 40% and 30% sucrose. After 2h centrifugation at 26000 rpm, the VLPs were recovered from the interface of 80% and 40% sucrose layers.

*Plaque assays:* Infectious Ebola and Marburg virions were enumerated using a standard plaque assay as previously described (Hevey et al., 1998, supra). Briefly, culture supernatants were serially diluted in EMEM. 100 ul of each dilution were added to wells of Vero-E6 cells in duplicate. Virus was allowed to adsorb for 50 minutes. Wells were then overlaid with 1X EBME and 0.5% agarose. Plates were incubated at 37°C, 5% CO₂ at which time a second overlay of 1X EBME/0.5% agarose and 20% neutral red was added to each well, incubated for additional 24 hours and plaques were counted.

*Cell staining and confocal microscopy:* 293T cells (human epithelial kidney cells, ATCC) were stained with indicated antibodies to viral proteins followed by Alexa-647 conjugated secondary antibodies (Molecular Probes, Eugene, Oregon). Rafts were visualized by staining of GM1 with Alexa-488 conjugated CTB and in some experiments with rhodamin-conjugated CTB (Figure 2B). Staining was performed on live cells on ice for 20 minutes. Cells were then washed with PBS, fixed in 3% paraformaldehyde, washed and mounted on microscopy slides. Images were collected using the BioRad (Hemel Hempstead, UK) Radiance 2000 system attached to a Nikon (Melville, NY) E600 microscope. Alexa-488 immunostain was excited using 488 nm light from a Krypton-Argon laser and the emitted light was passed through an HQ515/30 filter. Fluorescence from the Alexa-647 dye was excited by 637 nm light from a red diode laser and collected after passing through an HQ660LP emission filter. The lasers were programmed to scan over successive focal planes (0.25-0.5 um intervals) at 50 lines per sec. Lasersharp software was used to control the confocal system and to reconstruct individual focal planes into 3-dimensional renderings.

*Electron microscopy:* Portions of particulate material were applied to 300-mesh, nickel electron microscopy grids pre-coated with formvar and carbon, treated with 1% glutaraldehyde in PBS for 10 min, rinsed in distilled water, and negatively stained with 1% uranyl acetate. For immunoelectron microscopy, fractions were processed as previously described for fluid specimens (Geisbert and Jahrling, 1995, Virus Res. 39, 129). Briefly, fractions were applied to grids and immersed for 45 min in dilutions of monoclonal antibodies against EBOV GP. Normal mouse ascetic fluid was tested in parallel. Grids were washed with the TRIS buffer and incubated for 45 min with goat anti-mouse IgG labeled with 10 nm gold spheres (Ted Pella Inc. Redding, California). Grids were washed in PBS, and fixed in 1% glutaraldehyde. After fixation, grids were rinsed in drops of distilled water and negatively stained with 1% uranyl acetate. For pre-embedment staining, cells were stained with anti-Ebola GP mAb followed by gold-anti-mouse Ab, fixed with 2% glutaraldehyde in Millonig's buffer (pH7.4) for 1h and post-fixed in 1% uranylacetate, dehydrated and embedded in POLY/BED 812 resin (Polysciences, Warrington, PA). Resin was allowed to polymerize for 16h at 60°C, Ultrathin sections (~80nm) were cut, placed on 200-mesh copper electron microscopy grids and negatively stained. Stained grids were examined with a JEOL 1200 EX transmission electron microscope at 80 kV.

### Example 1

### Association of filovirus glycoproteins with lipid rafts:

Targeting of membrane-spanning proteins to lipid rafts is commonly governed by dual acylation of cysteine residues at the cytosolic end of the transmembrane domains (Rousso et al, 2000, supra; Zhang et al., 1998, Immunity 9, 239). The filovirus envelope glycoproteins (GP) contain such acylation signals in their transmembrane domains (Feldmann and Klenk, 1996, supra) and palmitoylation of Ebola GP has been recently reported (Ito et al., 2001, J. virol. 75, 1576). By transient expression of the filovirus envelope glycoproteins in 293T cells and subsequent extraction of rafts by sucrose gradient ultracentrifugation (Aman and Ravichandran, 2000, supra), we examined whether these glycoproteins localize to lipid rafts. As shown in figure 1 (A and B), a significant fraction of Ebola and Marburg GPs were found to reside in rafts. In contrast, an Ebola GP, mutated at cysteine residues 670 and 672 (Ebo-GP_{C670/672A}), the putative palmitoylation sites, failed to localize to the rafts (Figure 1B). Lipid rafts are highly enriched in ganglioside M1 (GM1) which can be detected by its specific binding to cholera toxin B (CTB) (Harder et al., 1998, J. Cell biol. 141, 929; Heyningen, S. V., 1974, Science 183, 656). As a control for the quality of raft preparations, we analyzed the soluble and raft fractions for the presence of GM1 by spot blots using HRP-conjugated CTB and demonstrated that GM1 was exclusively found in the raft fractions (Figure 1A and B, lower panels). The association of GP with detergent insoluble fraction was dependent on cholesterol since pre-treatment with methyl-β-cyclodextrin (MβCD), a drug that depletes the membrane from cholesterol (Christian et al., 1997, J. Lipid Res. 38, 2264), resulted in almost compete removal of Ebola GP from rafts (Figure 1C, upper panel). As a further control, we showed that transferrin receptor (TrfR), a molecule excluded from rafts (Harder et al., 1998, supra), was only found in the soluble fraction (Figure 1C, lower panel). To confirm the raft localization of Ebola and Marburg GP on intact cells, we also performed confocal laser microscopy on 293T cells that were transfected with Ebola or Marburg GP and co-stained with anti-GP antibodies and CTB. As shown in Figure 2A, a substantial portion of both of the glycoproteins were found to colocalize with GM1 in large patches on the plasma membrane, confirming the raft association of both glycoproteins on intact cells. Movies visualizing 25 sections through the cells, as well as three-dimensional (3-D) reconstruction of the cells by compiling data from these sections are available as supplemental data on the web (web movies 1 and 2). Confocal microscopy again showed that the membrane domains visualized by CTB staining were devoid of the raft excluded TrfR (Figure 2B).

### Example 2

### Filoviral proteins associate with lipid rafts in cells infected with live virus:

Two of the primary target cells of filoviruses are monocyte/macrophages and hepatocytes (Feldman and Klenk, 1996, supra). Thus, to examine the localization of EBOV and MBGV proteins with respect to lipid rafts during infection with live virus, primary human monocytes, HepG2 hepatocytes, and also Vero-E6 cells (commonly used to propagate filoviruses) were used as target cells. Human monocytes were infected with the Musoke strain of MBGV, after 24h detergent-insoluble and detergent-soluble fractions were separated by centrifugation (Rousso et al., 2000, supra). As shown in Figure 3A, a major fraction of viral proteins was detected in the detergent-insoluble fraction (I) 24 hours after infection. We then performed similar experiments with HepG2 cells, infected with EBOV-Zaire95 and prepared lipid rafts by sucrose gradient ultracentrifugation. Similar to Marburg, Ebola VP40 and GP were detected mainly in lipid rafts 24h after infection of HepG2 hepatocytes (Figure 3B). To further confirm the accumulation of filovirus proteins in lipid rafts in intact cells, Vero-E6 cells, infected with EBOV, were fixed, irradiated and costained with anti-Ebola antibody and CTB. As shown in Figure 3C, we observed a striking colocalization of viral proteins with the lipid rafts in intact Ebola-infected cells (see also web movies 5 and 6), suggesting that viral proteins assemble at lipid rafts during the course of viral replication.

### Example 3

### Ebola and Marburg virions incorporate the raft molecule GM1 during budding:

To determine whether the virus was released through lipid rafts, we analyzed EBOV from culture supernatants of infected cells for the presence of the raft marker GM1. Enveloped viruses bud as virions surrounded by the portion of the plasma membrane at which assembly takes place (Simons and Garoff, 1980, J. Gen. Virol. 50, 1). Release of virions through lipid rafts would therefore result in incorporation of raft-associated molecules in the viral envelope, thus identifying virus budding from the rafts. As shown in Figure 4A, EBOV immunoprecipitated with anti-Ebola GP antibody from supernatant of infected Vero-E6 cells contained readily detectable levels of GM1. We also analyzed inactivated Marburg virus that had been purified by ultracentrifugation for the incorporation of GM1 and demonstrated that GM1 was detectable in MBGV (Figure 4B, lower panel). In contrast, the raft-excluded protein TrfR was not incorporated in Marburg virions (Figure 4B, middle panel). Taken together, these data strongly suggested that both viruses exit cells through lipid rafts.

### Example 4

### Release of GM1-containing particles by ectopic expression of Ebola proteins:

To further test the hypothesis that filoviruses assemble and bud via lipid rafts, we transiently expressed viral proteins and searched for GM1-containing virus-like particles (VLPs). Several viral proteins have been shown to support the formation of VLPs (Porter et al., 1996, J. Virol. 70, 2643; Delchamber et al., 1989, EMBO J. 8, 2753; Thomsen et al., 1992, J. Gen. Virol. 73, 1819). In transfected 293T cells, Ebola GPwt, GP_{C670/672A}, and VP40 were readily detected in cell lysates when each protein was expressed individually (Figure 5A, panels 1 and 3; lanes 2,3,4). However, when VP40 and GP were coexpressed, little GP and almost no VP40 were found associated with the cells 60 hours after transfection (Figure 5A, panels 1 and 3; lane 5). To examine the viral proteins released from the cells, culture supernatants were cleared of cells, and particulate material was purified by ultracentrifugation over a 30% sucrose cushion. As shown in Figure 5A (panels 2 and 4; lanes 2-4), large amounts of GPwt and lesser quantities of GP_{C670/672A} or VP40 were detested in the particulate material from the supernatants of singly transfected cells. Interestingly, coexpression of GPwt and VP40, directed the majority of both proteins into the supernatant (Figure 5A, panels 2 and 4, lane 5). Next, we tested if the released particles incorporated the raft-associated molecule GM1. Anti-Ebo-GP immunoprecipitates from the supernatants of the cells transfected with GPwt or GPwt+VP40, but not GP_{C670/672A}, contained GM1 (Figure 5A, panel 5), suggesting that the release of these particles takes place through the rafts. We performed a second step of purification on these particles using a sucrose step gradient to separate the virus-like particles from the cell debris. The low density fraction floating between 40% and 80% sucrose was then analyzed by Western blot. As shown in Figure 5B, these particles contained GM1 but totally excluded transferrin receptor, further confirming the release of particles through lipid rafts.

### Example 5

*Particles formed by EBOV GP and VP40 display the morphological characteristics of Ebola virus:*

We determined the composition and morphology of these particles by examination of the purified particulate material using electron microscopy. Interestingly, most of the particles obtained from the supernatants of the cells cotransfected with GPwt and VP40 displayed a filamentous morphology strikingly similar to filoviruses (Figure 6A and B) (Geisbert and Jahrling, 1995, supra; Murphy et al., 1978, Ebola and Marburg virus morphology and taxonomy. 1st edition. S.R. Pattyn, editor. Elsevier, Amsterdam, pp. 1-61). In contrast, the material obtained from cells transfected with GPwt, GP_{C670/672A} or VP40 only contained small quantities of membrane fragments, likely released during cell death (data not shown). The virus-like particles (VLPs) generated by GP and VP40 were released at a high efficiency. Typically, we achieve a titer of 0.5-1.0x10⁶ VLPs/ml 2-3 days after transfection. The VLPs have a diameter of 50-70 nm and are 1-2 um in length (Figure 6). This is similar to the length range of Ebola virions found in cell culture supernatants after *in vitro* infection (Geisbert and Jahrling, 1995, supra). The shorter diameter of VLPs (as compared to 80 nm for EBOV) may be due to the lack of ribonucleoprotein complex. We observed all types of morphologies described for filoviruses such as branched, rod-, U- and 6-shaped forms (Feldman and Klenk, 1996, supra; Geisbert and Jahrling, 1995, supra) among these particles (Figure 6). In addition, the VLPs were coated with 5-10 nm surface projections or "spikes" (Figure 6), characteristic for EBOV (Feldman and Klenk, 1996, supra; Geisbert and Jahrling, 1995, supra). Immunogold staining of the VLPs with anti-Ebola GP antibodies demonstrated the identity of the spikes on the surface of the particles as Ebola glycoprotein (Figure 6 B). To visualize the process of the release of the VLPs, cells transfected with GP and VP40 were analyzed by electron microscopy after pre-embedment immunogold staining. Figure 6C shows a typical site of VLP release, where a large number of particles that stain for GP exit through a small region of the plasma membrane (indicated by arrows). These sites of VLP release have an average diameter of about 1 um. Given the incorporation of GM1 in the VLPs (Figure 5) these particle-releasing platforms most likely represent coalesced lipid raft domains.

### Example 6

### Entry of EBOV is dependent on the integrity of lipid rafts

Having established a critical role for lipid rafts in virus release, we sought to investigate if filoviruses utilize the same gateway for entry. To examine the role of lipid rafts in filovirus entry, the effects of raft-disrupting agents filipin and nystatin on Ebola infection were explored. Brief treatment of cells with filipin (0.2 ug/ml, 30 minutes) prior to infection resulted in a significant inhibition of EBOV infection evident by reduced viral titer 48 hour post infection (Figure 7). Similar results were also obtained with another cholesterol-destabilizing agent nystatin (Figure7). This effect was not due to a general cytotoxic effect by the drugs as cells were shown to be viable by trypan blue exclusion (data not shown). To rule out the possibility of a persistent effect of this brief drug treatment on the viral replication, we let an aliquot of the cells recover in medium (for 4h) after filipin treatment before infecting them with EBOV. As shown in Figure 7 (Filipin → recovery), these cells could produce large amounts of virus, ruling out the possibility of late effects of the drug on viral replication. In fact, in cells recovered from raft disruption the infection was even more efficient. This might be due to a synchronizing effect by reorganization of the microdomains resulting in a more efficient entry of the virus into a larger number of cells. We also considered the possibility that raft disruption may interfere with virus attachment rather than entry. However, titering of the virus recovered after the 50 minute binding showed that same amount of EBOV had bound to both treated and control cells (data not shown). Taken together, these data suggest that lipid rafts play a critical role in the entry stage of Ebola infection.

### Example 7

### Marburg VLP production

While both EBOV and MBGV appear to utilize the localization within lipid raft microdomains for viral assembly, other differences seem to exist between the two viruses in their replication mechanism. Ebola VP40 has been reported to be mainly localized to the plasma membrane (Ruigrok et al, 2000, J. Mol. Biol., 300(1):103-12) whereas Marburg VP40 has been shown to associate with late endosomes and multivesicular bodies (Kolesnikova et al, 2002, J Virol. 76(4):1825-38). Thus, it was not entirely clear whether VLPs could be formed in a similar manner for MBGV and if they would retain similar structure and morphology to the live virus.

In order to assess the ability of MBGV proteins to form VLPs, 293T cells were transfected with cDNAs encoding MBGV-Musoke GP as well as VP40 using lipofectamin-2000 according to manufacturer's instructions (Invitrogen, Carlsbad, CA). Cell supernatants were harvested after 48h and subjected to immunoprecipitation with mAb to Marburg GP and anti-mouse coated magnetic beads (Dynal, Lake Success, NY). Immunoprecipitates were washed with PBS and analyzed by immunoblotting. VP40 was coimmunoprecipitated with GP in supernatants of cells transected with both GP and VP40 (data not shown), suggesting that both proteins are released in a complex. The particulate materials was purified from the supernatants by sucrose gradient ultracentrifugation as described. Particulate material recovered from both the 10/40% and 40/60% interfaces was analyzed by Western blot using MBGV anti-GP and anti-VP40 specific antibodies. Western blot analysis indicates the presence of both viral proteins found in the 40% and 60% VLP fractions, suggesting that particles containing the viral proteins have a broad range of density (data not shown).

To determine if this particulate material in fact contains VLPs we analyzed the particles by electron microscopy. Structures similar to live virus were seen in both the 40% or 60% sucrose fractions purified from supernatants of GP/VP40 expressing cells (data not shown). Immunogold staining of the VLPs with MBGV anti-GP antibodies indicated the presence of glycoprotein spikes on the surface of the particles (data not shown). Taken together these data clearly indicate that, similar to Ebola virus, VLPs can be generated by coexpression of Marburg virus matrix and glycoproteins.

While in the case of HIV the raft localization is governed by myristylation of the matrix protein gag, no such signals are present in filoviral VP40. In contrast, raft localization of filoviral proteins seems to be driven by the glycoprotein that contains two palmitoylation sites at the end of its transmembrane domain (Ito et al., 2001, J Virol. 75(3):1576-80). These sites are essential for both raft localization as well as VLP release. The requirement for co-expression of GP for efficient release of VLPs suggests that GP may be facilitating this process by recruiting the assembly complex into raft microdomains. However, it is possible that other structural elements in GP, beside raft association signals, are also needed for the proper coordination of VP40 molecules to form the filamentous structure. VLPs represent an excellent safe and surrogate model for such structure function studies.

The addition of a vector encoding the nucleoprotein NP to the original transfection protocol also produces VLPs in a similar manner to GP + VP40. The sequence of Marburg NP is deposited in accession # NC_001608 with protein ID number: 042025.1. Western blot analysis of VLPs and immunoprecipitations confirm the presence of NP (data not shown). This suggests co-association of the proteins indicating the potential for filovirus like structures. This indicates that additional MGBV proteins may be incorporated into the structure thereby expanding the viral proteins which may serve as immunogens.

### Example 8

### Immunogenicity in mice.

The glycoprotein of filoviruses is the only protein expressed on the viral surface and is believed to be the main immunogenic determinant (Feldman and Klenk, 1996, supra). Delivery of Ebola GP as a DNA vaccine has been shown to protect mice from lethal challenge (Vanderzanden et al, 1998, Virology 246(1):134-44). Adenovirus mediated gene transfer of Ebola GP was also protective in non-human primates at least after low challenge dose (Sullivan et al, 2000, Nature 408 (6812) : 605-9). In addition, VP40 can provide some level of protective immune response in certain mouse strains (Wilson et al, Virology. 2001 Aug 1;286(2) :384-90). The filovirus like particles express both GP and VP40 in a filamentous structure strikingly similar to authentic viruses. These properties suggest that VLPs may be excellent vaccine candidates. Several other VLPs have been shown to be capable of triggering both arms of the immune system and protect against live virus challenge (Furumoto et al, 2002, J Med Invert. 49 (3-4) :124-33; Peters BS: Vaccine. 2001, 20(5-6):688-705). Therefore, we sought to examine the immunogenicity of EBOV and MBGV VLPs.

*EBOV VLPs protect mice against challenge with mouse-adapted EBOV.* To assess whether the EBOV VLPs could induce protection against infection with Ebola, mice were immunized three times intraperitoneally with 40 ug of VLPs and then challenged with mouse-adapted Ebola virus 3 weeks following the last immunization. Mice immunized with EBOV VLPs developed high titers of EBOV-specific antibodies, as determined by ELISA (Figure 8a). Additionally, serum from EBOV VLP-immunized mice was able to neutralize EBOV infection of VeroE6 cells (Figure 8b). Following challenge with 300 pfu of EBOV, ten of ten mice immunized with EBOV VLPs survived, while mice immunized with inactivated EBOV or MBGV had only low survival (Figure 9). One of ten naïve mice survived following EBOV challenge (Figure 9). The viral load of the VLP-immunized mice (n=10) was 20 ± 42 pfu at 7 days following challenge.

### Discussion

Our findings demonstrate that filoviruses utilize lipid rafts as a platform for budding from the cells. We documented this phenomenon in reconstruction experiments and in the process of live virus infections. Both after transient expression of filovirus glycoproteins as well as in EBOV and MBGV infected cells, we observed large patches of envelope glycoproteins in association with lipid rafts (Figures 1,2, and 3). Our results also demonstrate that the released virions incorporate the raft-associated molecule GM1, but not transferrin receptor, a protein excluded from lipid rafts (Harder et al., 1998, supra). Using electron microscopy on cells transfected with Ebola GP and matrix protein VP40, we also demonstrate the site of release of Ebola-like particles to be localized in a small area of the plasma membrane about 1um wide (Figure 6C). Therefore, such patches of rafts appear to represent the site of filovirus assembly and budding. Electron microscopic studies show that virus budding at the plasma membrane requires an accumulation of viral components including nucleocapsid, matrix and envelope glycoprotein in an orchestrated manner, concurrent with structural changes in the plasma membrane (Dubois-Delcq and Reese, 1975, J. Cell Biol. 67, 551). This process is dependent on a precise coordination of the involved components (Garoff et al., 1998, Microbiol. Mol. Biol. Rev. 62, 1171). Thus, compartmentalization of viral assembly in a specialized microdomain, such as rafts, with its ordered architecture and selective array of molecules may increase the efficiency of virus budding and decrease the frequency of release of defective, noninfectious particles.

Besides acting as a coordination site for viral assembly, rafts may have a profound impact on viral pathogenicity as well as host immune response to viruses. Transfer of the incorporated molecules with signaling capabilities into newly infected cells may affect the intracellular biochemical processes in favor of a more efficient viral replication. Furthermore, selective enrichment of certain proteins such as adhesion molecules can affect the efficiency of viral entry and possibly virus tropism. Incorporation of GPI-anchored proteins in the viral envelope such as inhibitors of complement pathway CD55 and CD59, which have been detected in HIV virions (Saifuddin et al., 1997, J. Gen. Virol. 78, 1907), may help the virus evade the complement-mediated lysis. An important aspect of our study is the generation of genome-free filovirus-like particles. Our biochemical data show that the VLPs incorporate both Ebola GP and matrix protein VP40, as well as raft-associated ganglioside M1, similar to the results obtained with live virus infections (Figure 4). A striking morphological similarity between these VLPs and live filoviruses was observed in electron microscopic studies (Figure 6). These findings have several important implications. While several viral matrix proteins support the formation of VLPs, Ebola VP40 seems to be unique in that it requires the expression of envelope glycoprotein for efficient formation of particles. Recently, Timmins *et al* reported that a small fraction of transfected VP40 can be detected in culture supernatants in association with filamentous particles (Timmins et al., 2001, Virology 283, 1). While we detected VP40 in the supernatants of
transfected 293T cells, electron microscopic analysis revealed that the protein was associated with unstructured membrane fragments. In multiple experiments, filamentous particles were only observe when both VP40 and GP were concurrently expressed. These findings imply that the driving force for the assembly and release of EBOV may be the interaction between GP and matrix protein, as suggested previously (Feldman and Klenk, 1996, supra). Ebola VP40 has an N-terminal and a C-terminal domain, the latter being involved in membrane localization (Dessen et al., 2000, EMBO J. 19, 4228). Removal of most of the C-terminal domain induces hexamerization of the protein, the multimeric form believed to be involved in viral assembly (Ruigrok et al., 2000, J. Mol. Biol. 300, 103). While our data show that the majority of VP40 is membrane associated, we were unable to detect VP40 in the rafts when expressed independently (data not shown). Our attempts to detect VP40 in the lipid rafts in the presence of GP was hampered by the efficient release of the proteins in the supernatants resulting in hardly detectable cellular levels of VP40 (Figure 3). However, given the incorporation of GM1 in the VP40-containing VLPs, it is reasonable to speculate that a transient association of VP40 with lipid rafts takes place in the cells. It is possible that association of VP40 with GP drivels VP40 into the rafts. Since a fraction of GP is outside the rafts (Figure 1), probably in a dynamic exchange with the rafts, this pool of GP might be involved in the initial interaction with VP40. This interaction and subsequent movement to the rafts may, at the same time, induce a conformational change in VP40 resulting in dissociation of the C-terminal domain from the non-raft membrane and thus removing the constraints on the formation of VP40 hexamers required for viral assembly. Detailed studies are underway to test this model. In this regard, the successful generation of VLPs by ectopic expression of viral proteins provides a safe approach for the study of the steps involved in filovirus assembly and budding without the restrictions of biosafety level-4 laboratories.

VLPs could be an excellent vehicle for antigen delivery, thus useful as a vaccination strategy (Johnson and Chiu, 2000, Curr. Opin. Struct. Biol. 10, 229; Wagner et al., 1999, Vaccine 17, 1705). Different types of recombinant HIV-1 virus-like particles have been shown to not only trigger the induction of neutralizing antibodies but also induce HIV-specific CD8⁺ CTL responses in preclinical studies (Wagner et al., 1999, supra). Therefore, VLPs are capable of mobilizing different arms of the adaptive immune system. Given the importance of both cellular and humoral immune response for protection against Ebola (Wilson et al., 2000, supra; Wilson and Hart 2001, J. Virol. 75, 2660), filovirus-based VLPs, alone or in combination with DNA vaccination, may represent a good vaccine candidate. We are currently investigating the capability of Ebola and Marburg VLPs to elicit an immune response. Another potential use of VLPs is in the delivery of foreign antigens. Parvovirus-like particles have been engineered to express foreign polypeptides, resulting in the production of large quantities of highly immunogenic peptides, and to induce strong antibody, T helper cell, and CTL response (Wagner al., 1999, supra). Given the compartmentalized release of VLPs through rafts, artificial targeting of antigens to lipid rafts by introduction of dual acylation signals may result in their enrichment in filovirus-based VLPs, providing a potential novel strategy for delivery of a variety of antigens.

VLPs are also valuable research tools. Mutational analysis of the proteins involved in filovirus release can be performed using VLP formation as a quick readout. Our VLPs express the envelope glycoprotein in addition to the matrix protein and can therefore be also used for detailed study of the steps involved in the fusion and entry of EBOV and MBGV by circumventing the restrictions of working under biosafety level-4 conditions.

Most enveloped viruses use a specific interaction between their glycoproteins and cell surface receptors to initiate the attachment to the cells and subsequent fusion. Organization of viral preceptors in the ordered environment of lipid rafts may facilitate the virus binding through its multimeric glycoprotein, promote lateral assemblies at the plasma membrane required for productive infections, concentrate the necessary cytosolic and cytoskeletal components, and enhance the fusion process by providing energetically favorable conditions. It is intriguing that the HIV receptor CD4 (Xavier et al., 1998, supra), its coreceptor CXCR4 (Manes et al., 2000, supra), as well as molecules favoring HIV infection such as glycosphingolipids (Simons and Ikonen, 1997, supra; Hug et al., 2000, J. Virol. 74, 6377), and CD44 (Viola et al., 1999, supra; Dukes et al., 1995, J. Virol. 69, 4000) all reside in lipid rafts. Our data suggest that filoviruses use lipid rafts as a gateway for the entry into cells. This may relate to the presence of the filovirus receptor(s) in these microdomains. Recently, it has been demonstrated that folate receptor-α can function as a cellular receptor for filoviruses (Chan et al., 2001, Cell 106, 117). Interestingly, folate receptor-α is a GPI-anchored protein shown to reside in the rafts (Nichols et al., 2001, J. Cell Biol. 153, 529). Thus, rafts may be crucial for viral entry by concentrating the receptor for filovirus glycoproteins. Our finding that disruption of lipid rafts can interfere with filovirus entry suggests that the integrity of these compartments or their molecular components may be potential therapeutic targets against Ebola and Marburg infections. Further characterization of the raft composition during host-virus interaction, for instance by proteomic analysis, will help to identify such potential targets.

In summary, our findings shed new light on the molecular mechanisms involved in filovirus entry as well as assembly and budding. Much deeper understanding of these mechanisms is needed for successful design of efficacious therapeutic and vaccination strategies. However, identification of rafts as a gateway for cellular trafficking of Ebola and Marburg viruses and generation of Ebola VLPs are important steps toward this goal.

## Claims

1. A filovirus virus like particle (VLP) comprising filovirus envelope glycoprotein, GP, and filovirus matrix protein, VP40.

2. A VLP according to claim 1, further comprising a filovirus nucleoprotein (NP).

3. A VLP according to claim 1 or 2 wherein said filovirus is chosen from the group consisting of Ebola and Marburg.

4. A VLP according to claim 3, wherein said GP comprises a portion from Ebola GP and a portion from Marburg GP.

5. A VLP according to claim 3 or 4, wherein said VP40 comprises a portion from Ebola VP40 and a portion from Marburg VP40.

6. A method of producing a filovirus virus like particle (VLP) comprising expressing in a cell a polynucleotide encoding filovirus envelope glycoprotein, GP, and filovirus matrix protein, VP40 such that said polynucleotide is expressed and said VLP is produced.

7. A method according to claim 6, wherein said polynucleotide further encodes filovirus nucleoprotein (NP)

8. A method according to claim 6 or 7 wherein said filovirus is chosen from the group consisting of Ebola and Marburg.

9. A method according to claim 8, wherein said polynucleotide encoding GP comprises a portion from Ebola GP and a portion from Marburg GP.

10. A method according to claim 8 or 9, wherein said polynucleotide encoding VP40 comprises a portion from Ebola VP40 and a portion from Marburg VP40.

11. A method according to any one of claims 6 to 10, wherein said cell is a mammalian cell.

12. A method according to any one of claims 6 to 10, wherein said cell is an insect cell.

13. A filovirus virus like particle (VLP) of any one of claims 1 to 5 or obtainable by a method according to any one of claims 6 to 12 for use in a therapeutic or diagnostic method for introducing an agent into a cell or for delivering a foreign antigen *in vivo,* wherein said agent or foreign antigen is packaged or contained in said VLP.

14. A method for testing an agent involved in filovirus budding, comprising introducing said agent to a cultured cell producing a filovirus virus like particle (VLP) of any one of claims 1 to 5 or obtainable by a method according to any one of claims 6 to 12 and monitoring the presence or absence of a change in the budding of VLP as compared to a control by measuring VLPs in supernatant of said cultured cell, wherein a reduction or increase in the number of VLP in the supernatant indicates a negative or positive agent, respectively, on filovirus budding.

15. The method according to claim 14 wherein said filovirus is chosen from the group consisting of Ebola and Marburg.

16. A method for detecting Ebola virus infection or Marburg virus infection comprising contacting a sample from the subject suspected of having Ebola virus infection or Marburg virus infection with a virus like particle (VLP) according to any one of claims 3 to 5 and detecting the presence or absence of an infection by detecting the presence or absence of a complex formed between the VLP and antibodies specific therefor in said sample.

17. A kit for the detection of Ebola virus or Marburg virus infection comprising virus like particles (VLPs) according to any one of claims 3 to 5.

18. A host cell transformed with a recombinant polynucleotide encoding filovirus envelope glycoprotein (GP) and Filovirus Matrix protein (VP40) and comprising a VLP as defined in any one of claims 1 to 5.

19. The host cell according to claim 18, wherein said VLP is obtainable by the method of any one of claims 8 to 12.

20. The host cell according to claim 18 or 19 wherein said cell comprises a mammalian cell or an insect cell.

21. An immunogenic composition comprising, in a physiologically acceptable vehicle, Ebola virus like particles (VLPs) or Marburg virus like particles (VLPs) of any one of claims 3 to 5.

22. The immunogenic composition according to claim 21 which further comprises an adjuvant to enhance the immune response.

23. The immunogenic composition according to claim 21 or 22, wherein said VLPs are obtainable by a method according to any one of claims 6 to 12.

24. Use of Ebola virus like particles (VLPs) or Marburg virus like particles (VLPs) according to any one of claims 3 to 5 or obtainable by a method according to any one of claims 6 to 12 in the manufacture of a medicament for stimulating an Ebola or Marburg virus specific immune response.

## Patentansprüche

1. Filovirus-Virus-ähnliches Partikel (VLP), das Filovirus-Hüllglycoprotein, GP, und Filovirus-Matrixprotein, VP40, umfasst.

2. VLP gemäß Anspruch 1, das weiter ein Filovirus-Nucleoprotein (NP) umfasst.

3. VLP gemäß Anspruch 1 oder 2, wobei der Filovirus ausgewählt ist aus der Gruppe, bestehend aus Ebola und Marburg.

4. VLP gemäß Anspruch 3, wobei das GP einen Abschnitt von Ebola-GP und einen Abschnitt von Marburg-GP umfasst.

5. VLP gemäß Anspruch 3 oder 4, wobei das VP40 einen Abschnitt von Ebola-VP40 und einen Abschnitt von Marburg VP40 umfasst.

6. Verfahren zur Herstellung eines Filovirus-Virus-ähnlichen Partikels (VLP), das das Exprimieren eines Polynucleotids, das Filovirus-Hüllglycoprotein, GP, und Filovirus-Matrixprotein, VP40, kodiert in einer Zelle umfasst, sodass das Polynucleotid exprimiert wird und das VLP hergestellt wird.

7. Verfahren gemäß Anspruch 6, wobei das Polynucleotid weiter Filovirus-Nucleoprotein (NP) kodiert.

8. Verfahren gemäß Anspruch 6 oder 7, wobei der Filovirus ausgewählt ist aus der Gruppe, bestehend aus Ebola und Marburg.

9. Verfahren gemäß Anspruch 8, wobei das Polynucleotid, das GP kodiert, einen Abschnitt von Ebola-GP und einen Abschnitt von Marburg-GP umfasst.

10. Verfahren gemäß Anspruch 8 oder 9, wobei das Polynucleotid, das VP40 kodiert, einen Abschnitt von Ebola-VP40 und einen Abschnitt von Marburg-VP40 umfasst.

11. Verfahren gemäß einem der Ansprüche 6 bis 10, wobei die Zelle eine Säugerzelle ist.

12. Verfahren gemäß einem der Ansprüche 6 bis 10, wobei die Zelle eine Insektenzelle ist.

13. Filovirus-Virus-ähnliches Partikel (VLP) gemäß einem der Ansprüche 1 bis 5, oder erhältlich durch ein Verfahren gemäß einem der Ansprüche 6 bis 12, zur Verwendung in einem therapeutischen oder diagnostischen Verfahren zur Einführung eines Mittels in eine Zelle, oder zur Abgabe eines fremden Antigens *in vivo,* wobei das Mittel oder das fremde Antigen in das/in dem VLP verpackt oder enthalten ist.

14. Verfahren zur Testung eines Mittels, das in die Filovirus-Knospung involviert ist, umfassend das Einführen des Mittels in eine kultivierte Zelle, Herstellen eines Filovirus-Virus-ähnlichen Partikels (VLP) gemäß einem der Ansprüche 1 bis 5, oder erhältlich durch ein Verfahren gemäß einem der Ansprüche 6 bis 12, und Überwachen der Anwesenheit oder Abwesenheit einer Veränderung der Knospung von VLP im Vergleich zu einer Kontrolle durch Messen von VLPs im Überstand der kultivierten Zelle, wobei eine Reduktion oder Zunahme in der Anzahl an VLP in dem Überstand ein negatives bzw. positives Mittel für Filovirus-Knospung anzeigt.

15. Verfahren gemäß Anspruch 14, wobei der Filovirus ausgewählt ist aus der Gruppe, bestehend aus Ebola und Marburg.

16. Verfahren zum Nachweis von Ebola-Virusinfektion oder Marburg-Virusinfektion, umfassend das Inkontaktbringen einer Probe von dem Subjekt, von dem angenommmen wird, dass es Ebola-Virusinfektion oder Marburg-Virusinfektion hat, mit einem Virus-ähnlichen Partikel (VLP) gemäß einem der Ansprüche 3 bis 5 und Nachweisen der Anwesenheit oder Abwesenheit einer Infektion durch Nachweisen der Anwesenheit oder Abwesenheit eines Komplexes, der zwischen dem VLP und Antikörpern, die spezifisch dafür sind, in der Probe gebildet wurde.

17. Kit zum Nachweis von Ebola-Virus- oder Marburg-Virusinfektion, der Virus-ähnliche Partikel (VLPs) gemäß einem der Ansprüche 3 bis 5 umfasst.

18. Wirtszelle, die mit einem rekombinanten Polynucleotid transformiert ist, das Filovirus-Hüllglycoprotein (GP) und Filovirus-Matrixprotein (VP40) kodiert und die ein VLP, wie definiert in einem der Ansprüche 1 bis 5, umfasst.

19. Wirtszelle gemäß Anspruch 18, wobei das VLP durch das Verfahren gemäß einem der Ansprüche 8 bis 12 erhältlich ist.

20. Wirtszelle gemäß Anspruch 18 oder 19, wobei die Zelle eine Säugerzelle oder eine Insektenzelle umfasst.

21. Immunogene Zusammensetzung, die in einem physiologisch verträglichen Vehikel Ebola-Virus-ähnliche Partikel (VLPs) oder Marburg-Virus-ähnliche Partikel (VLPs) gemäß einem der Ansprüche 3 bis 5 umfasst.

22. Immunogene Zusammensetzung gemäß Anspruch 21, die weiter ein Adjuvans umfasst, um die Immunantwort zu verstärken.

23. Immunogene Zusammensetzung gemäß Anspruch 21 oder 22, wobei die VLPs durch ein Verfahren gemäß einem der Ansprüche 6 bis 12 erhältlich sind.

24. Verwendung von Ebola-Virus-ähnlichen Partikeln (VLPs) oder Marburg-Virus-ähnlichen Partikeln (VLPs) gemäß einem der Ansprüche 3 bis 5, oder erhältlich durch ein Verfahren gemäß einem der Ansprüche 6 bis 12, zur Herstellung eines Medikaments zur Stimulation einer Ebola oder Marburg virusspezifischen Immunantwort.

## Revendications

1. Particule VLP (particule "virus-like", ou pseudo-particule virale) de filovirus, comprenant une glycoprotéine d'enveloppe GP de filovirus et une protéine de matrice VP40 de filovirus.

2. Particule VLP conforme à la revendication 1, comprenant en outre une nucléoprotéine NP de filovirus.

3. Particule VLP conforme à la revendication 1 ou 2, ledit filovirus étant choisi dans l'ensemble formé par les virus Ebola et Marburg.

4. Particule VLP conforme à la revendication 3, dans laquelle ladite protéine GP comprend une partie provenant de la protéine GP d'Ebola et une partie provenant de la protéine GP de Marburg.

5. Particule VLP conforme à la revendication 3 ou 4, dans laquelle ladite protéine VP40 comprend une partie provenant de la protéine VP40 d'Ebola et une partie provenant de la protéine VP40 de Marburg.

6. Procédé de production d'une particule VLP (particule "virus-like") de filovirus, comportant le fait de faire s'exprimer, dans une cellule, un polynucléotide codant une glycoprotéine d'enveloppe GP de filovirus et une protéine de matrice VP40 de filovirus, de telle manière que ledit polynucléotide soit exprimé et que ladite particule VLP soit produite.

7. Procédé conforme à la revendication 6, dans lequel ledit polynucléotide code en outre une nucléoprotéine NP de filovirus.

8. Procédé conforme à la revendication 6 ou 7, ledit filovirus étant choisi dans l'ensemble formé par les virus Ebola et Marburg.

9. Procédé conforme à la revendication 8, dans lequel ledit polynucléotide codant une protéine GP comprend une partie codant une partie de la protéine GP d'Ebola et une partie codant une partie de la protéine GP de Marburg.

10. Procédé conforme à la revendication 8 ou 9, dans lequel ledit polynucléotide codant une protéine VP40 comprend une partie codant une partie de la protéine VP40 d'Ebola et une partie codant une partie de la protéine VP40 de Marburg.

11. Procédé conforme à l'une des revendications 6 à 10, dans lequel ladite cellule est une cellule de mammifère.

12. Procédé conforme à l'une des revendications 6 à 10, dans lequel ladite cellule est une cellule d'insecte.

13. Particule VLP (particule "virus-like", ou pseudo-particule virale) de filovirus, conforme à l'une des revendications 1 à 5 ou accessible par un procédé conforme à l'une des revendications 6 à 12, pour utilisation dans un procédé, à visée thérapeutique ou diagnostique, d'introduction d'un agent dans une cellule ou d'apport d'un antigène étranger *in vivo,* ledit agent ou antigène étranger étant emballé ou contenu dans ladite particule VLP.

14. Procédé permettant de tester un agent impliqué dans le bourgeonnement des filovirus, lequel procédé comporte le fait d'introduire ledit agent dans une culture de cellules produisant des particules VLP (particules "virus-like") de filovirus, conformes à l'une des revendications 1 à 5 ou accessibles par un procédé conforme à l'une des revendications 6 à 12, et le fait d'observer si oui ou non le bourgeonnement des particules VLP est modifié, en comparaison d'un témoin, en mesurant la quantité de particules VLP présentes dans le surnageant de ladite culture de cellules, étant entendu qu'une diminution ou une augmentation du nombre de particules VLP présentes dans le surnageant est un indice de ce que l'agent a un effet, respectivement négatif ou positif, sur le bourgeonnement des filovirus.

15. Procédé conforme à la revendication 14, ledit filovirus étant choisi dans l'ensemble formé par les virus Ebola et Marburg.

16. Procédé permettant de détecter une infection par le virus Ebola ou une infection par le virus Marburg, lequel procédé comporte le fait de mettre un échantillon, provenant d'un sujet suspecté d'être atteint d'une infection par le virus Ebola ou d'une infection par le virus Marburg, en contact avec des particules VLP (particules "virus-like") conformes à l'une des revendications 3 à 5, et le fait de détecter la présence ou l'absence d'une infection en observant, dans ledit échantillon, la présence ou l'absence de complexes formés entre les particules VLP et les anticorps spécifiques dirigés contre elles.

17. Trousse servant à détecter une infection par le virus Ebola ou une infection par le virus Marburg, laquelle trousse comprend des particules VLP (particules "virus-like") conformes à l'une des revendications 3 à 5.

18. Cellule hôte transformée avec un polynucléotide recombiné codant une glycoprotéine d'enveloppe GP de filovirus et une protéine de matrice VP40 de filovirus, et comprenant une particule VLP conforme à l'une des revendications 1 à 5.

19. Cellule hôte conforme à la revendication 18, pour laquelle ladite particule VLP est accessible par un procédé conforme à l'une des revendications 8 à 12.

20. Cellule hôte conforme à la revendication 18 ou 19, laquelle cellule est une cellule de mammifère ou une cellule d'insecte.

21. Composition immunogène comprenant, dans un véhicule physiologiquement admissible, des particules VLP (particules "virus-like") d'Ebola ou des particules VLP (particules "virus-like") de Marburg, conformes à l'une des revendications 3 à 5.

22. Composition immunogène conforme à la revendication 21, qui comporte en outre un adjuvant servant à renforcer la réponse immunitaire.

23. Composition immunogène conforme à la revendication 21 ou 22, pour laquelle lesdites particules VLP sont accessibles par un procédé conforme à l'une des revendications 6 à 12.

24. Utilisation de particules VLP (particules "virus-like") Ebola ou de particules VLP (particules "virus-like") de Marburg, conformes à l'une des revendications 3 à 5 ou accessibles par un procédé conforme à l'une des revendications 6 à 12, dans la fabrication d'un médicament destiné à stimuler une réponse immunitaire spécifique contre le virus Ebola ou le virus Marburg.
